(19) Europäisches Patentamt
European Patent Office
Office européen des brevets

(11) EP 1 743 523 A1

(12) **EUROPEAN PATENT APPLICATION**
published in accordance with Art. 158(3) EPC

(43) Date of publication:
**17.01.2007 Bulletin 2007/03**

(21) Application number: **05729372.2**

(22) Date of filing: **08.04.2005**

(51) Int Cl.:
***A01K 67/027*** *(2006.01)* ***A01K 67/02*** *(2006.01)*
***C12N 15/12*** *(2006.01)* ***C12N 15/65*** *(2006.01)*
***C12N 15/85*** *(2006.01)* ***G01N 33/50*** *(2006.01)*

(86) International application number:
**PCT/JP2005/007285**

(87) International publication number:
**WO 2005/096810 (20.10.2005 Gazette 2005/42)**

(84) Designated Contracting States:
**CH DE FR GB LI**

(30) Priority: **09.04.2004 JP 2004115646**

(71) Applicant: **University of Tsukuba**
**Tsukuba-shi, Ibaraki 305-8577 (JP)**

(72) Inventors:
- **SHIMANO, Hitoshi;**
**a-shi, Ibaraki 3058577; (JP)**
- **YAMADA, Nobuhiro;**
**a-shi, Ibaraki 3058577; (JP)**

(74) Representative: **Baldock, Sharon Claire**
**BOULT WADE TENNANT,**
**Verulam Gardens**
**70 Gray's Inn Road**
**London WC1X 8BT (GB)**

(54) **NONHUMAN TRANSGENIC ANIMAL AS TYPE 2 DIABETES MODEL**

(57) The invention provides a non-human transgenic animal as a model of type 2 diabetes manifesting a symptom of type 2 diabetes by excessive expression of the active SREBP-2 protein in pancreatic β-cells by introducing a recombinant DNA in which a DNA encoding the active SREBP-2 protein is disposed under the control of a promoter, and a method for screening therapeutic agents of diabetes using the transgenic animal.

EP 1 743 523 A1

SEQUENCE LISTING

<110> University of Tsukuba

<120> NONHUMAN TRANSGENIC ANIMAL AS TYPE 2 DIABETES MODEL

<130> G06-0074

<150> JP 2004-115646

<151> 2004-04-09

<160> 14

<210> 1

<211> 1141

<212> PRT

<213> primer

<400> 1

Met Asp Asp Ser Gly Glu Leu Gly Gly Leu Glu Thr Met Glu Thr Leu
1               5                   10                  15

Thr Glu Leu Gly Asp Glu Leu Thr Leu Gly Asp Ile Asp Glu Met Leu
            20                  25                  30

Gln Phe Val Ser Asn Gln Val Gly Glu Phe Pro Asp Leu Phe Ser Glu
        35                  40                  45

Gln Leu Cys Ser Ser Phe Pro Gly Ser Gly Gly Ser Gly Ser Ser Ser
    50                  55                  60

Gly Ser Ser Gly Ser Ser Ser Ser Ser Ser Asn Gly Arg Gly Ser Ser
65                  70                  75                  80

Ser Gly Ala Val Asp Pro Ser Val Gln Arg Ser Phe Thr Gln Val Thr
                85                  90                  95

Leu Pro Ser Phe Ser Pro Ser Ala Ala Ser Pro Gln Ala Pro Thr Leu

100 105 110

Gln Val Lys Val Ser Pro Thr Ser Val Pro Thr Thr Pro Arg Ala Thr

115 120 125

Pro Ile Leu Gln Pro Arg Pro Gln Pro Gln Pro Gln Pro Gln Thr Gln

130 135 140

Leu Gln Gln Gln Thr Val Met Ile Thr Pro Thr Phe Ser Thr Thr Pro

145 150 155 160

Gln Thr Arg Ile Ile Gln Gln Pro Leu Ile Tyr Gln Asn Ala Ala Thr

165 170 175

Ser Phe Gln Val Leu Gln Pro Gln Val Gln Ser Leu Val Thr Ser Ser

180 185 190

Gln Val Gln Pro Val Thr Ile Gln Gln Gln Val Gln Thr Val Gln Ala

195 200 205

Gln Arg Val Leu Thr Gln Thr Ala Asn Gly Thr Leu Gln Thr Leu Ala

210 215 220

Pro Ala Thr Val Gln Thr Val Ala Ala Pro Gln Val Gln Gln Val Pro

225 230 235 240

Val Leu Val Gln Pro Gln Ile Ile Lys Thr Asp Ser Leu Val Leu Thr

245 250 255

Thr Leu Lys Thr Asp Gly Ser Pro Val Met Ala Ala Val Gln Asn Pro

260 265 270

Ala Leu Thr Ala Leu Thr Thr Pro Ile Gln Thr Ala Ala Leu Gln Val

275 280 285

Pro Thr Leu Val Gly Ser Ser Gly Thr Ile Leu Thr Thr Met Pro Val

290 295 300

Met Met Gly Gln Glu Lys Val Pro Ile Lys Gln Val Pro Gly Gly Val

305 310 315 320

Lys Gln Leu Glu Pro Pro Lys Glu Gly Glu Arg Arg Thr Thr His Asn

325 330 335

Ile Ile Glu Lys Arg Tyr Arg Ser Ser Ile Asn Asp Lys Ile Ile Glu
340 345 350

Leu Lys Asp Leu Val Met Gly Thr Asp Ala Lys Met His Lys Ser Gly
355 360 365

Val Leu Arg Lys Ala Ile Asp Tyr Ile Lys Tyr Leu Gln Gln Val Asn
370 375 380

His Lys Leu Arg Gln Glu Asn Met Val Leu Lys Leu Ala Asn Gln Lys
385 390 395 400

Asn Lys Leu Leu Lys Gly Ile Asp Leu Gly Ser Leu Val Asp Asn Glu
405 410 415

Val Asp Leu Lys Ile Glu Asp Phe Asn Gln Asn Val Leu Leu Met Ser
420 425 430

Pro Pro Ala Ser Asp Ser Gly Ser Gln Ala Gly Phe Ser Pro Tyr Ser
435 440 445

Ile Asp Ser Glu Pro Gly Ser Pro Leu Leu Asp Asp Ala Lys Val Lys
450 455 460

Asp Glu Pro Asp Ser Pro Pro Val Ala Leu Gly Met Val Asp Arg Ser
465 470 475 480

Arg Ile Leu Leu Cys Val Leu Thr Phe Leu Cys Leu Ser Phe Asn Pro
485 490 495

Leu Thr Ser Leu Leu Gln Trp Gly Gly Ala His Asp Ser Asp Gln His
500 505 510

Pro His Ser Gly Ser Gly Arg Ser Val Leu Ser Phe Glu Ser Gly Ser
515 520 525

Gly Gly Trp Phe Asp Trp Met Met Pro Thr Leu Leu Leu Trp Leu Val
530 535 540

Asn Gly Val Ile Val Leu Ser Val Phe Val Lys Leu Leu Val His Gly
545 550 555 560

Glu Pro Val Ile Arg Pro His Ser Arg Ser Ser Val Thr Phe Trp Arg

565 570 575

His Arg Lys Gln Ala Asp Leu Asp Leu Ala Arg Gly Asp Phe Ala Ala

580 585 590

Ala Ala Ala Asn Leu Gln Thr Cys Leu Ala Val Leu Gly Arg Ala Leu

595 600 605

Pro Thr Ser Arg Leu Asp Leu Ala Cys Ser Leu Ser Trp Asn Val Ile

610 615 620

Arg Tyr Ser Leu Gln Lys Leu Arg Leu Val Arg Trp Leu Leu Lys Lys

625 630 635 640

Val Phe Gln Cys Arg Arg Ala Thr Pro Ala Thr Glu Ala Gly Phe Glu

645 650 655

Asp Glu Ala Lys Thr Ser Ala Arg Asp Ala Ala Leu Ala Tyr His Arg

660 665 670

Leu His Gln Leu His Ile Thr Gly Lys Leu Pro Ala Gly Ser Ala Cys

675 680 685

Ser Asp Val His Met Ala Leu Cys Ala Val Asn Leu Ala Glu Cys Ala

690 695 700

Glu Glu Lys Ile Pro Pro Ser Thr Leu Val Glu Ile His Leu Thr Ala

705 710 715 720

Ala Met Gly Leu Lys Thr Arg Cys Gly Gly Lys Leu Gly Phe Leu Ala

725 730 735

Ser Tyr Phe Leu Ser Arg Ala Gln Ser Leu Cys Gly Pro Glu His Ser

740 745 750

Ala Val Pro Asp Ser Leu Arg Trp Leu Cys His Pro Leu Gly Gln Lys

755 760 765

Phe Phe Met Glu Arg Ser Trp Ser Val Lys Ser Ala Ala Lys Glu Ser

770 775 780

Leu Tyr Cys Ala Gln Arg Asn Pro Ala Asp Pro Ile Ala Gln Val His

785 790 795 800

Gln Ala Phe Cys Lys Asn Leu Leu Glu Arg Ala Ile Glu Ser Leu Val
805 810 815
Lys Pro Gln Ala Lys Lys Lys Ala Gly Asp Gln Glu Glu Glu Ser Cys
820 825 830
Glu Phe Ser Ser Ala Leu Glu Tyr Leu Lys Leu Leu His Ser Phe Val
835 840 845
Asp Ser Val Gly Val Met Ser Pro Pro Leu Ser Arg Ser Ser Val Leu
850 855 860
Lys Ser Ala Leu Gly Pro Asp Ile Ile Cys Arg Trp Trp Thr Ser Ala
865 870 875 880
Ile Thr Val Ala Ile Ser Trp Leu Gln Gly Asp Asp Ala Ala Val Arg
885 890 895
Ser His Phe Thr Lys Val Glu Arg Ile Pro Lys Ala Leu Glu Val Thr
900 905 910
Glu Ser Pro Leu Val Lys Ala Ile Phe His Ala Cys Arg Ala Met His
915 920 925
Ala Ser Leu Pro Gly Lys Ala Asp Gly Gln Gln Ser Ser Phe Cys His
930 935 940
Cys Glu Arg Ala Ser Gly His Leu Trp Ser Ser Leu Asn Val Ser Gly
945 950 955 960
Gly Thr Ser Asp Pro Ala Leu Asn His Val Val Gln Leu Leu Thr Cys
965 970 975
Asp Leu Leu Leu Ser Leu Arg Thr Ala Leu Trp Gln Lys Gln Ala Ser
980 985 990
Ala Ser Gln Ala Val Gly Glu Thr Tyr His Ala Ser Gly Ala Glu Leu
995 1000 1005
Ala Gly Phe Gln Arg Asp Leu Gly Ser Leu Arg Arg Leu Ala His
1010 1015 1020
Ser Phe Arg Pro Ala Tyr Arg Lys Val Phe Leu His Glu Ala Thr

1025 1030 1035

Val Arg Leu Met Ala Gly Gly Ser Pro Thr Arg Thr His Gln Leu

1040 1045 1050

Leu Glu His Ser Leu Arg Arg Arg Thr Thr Gln Ser Thr Lys His

1055 1060 1065

Gly Glu Val Asp Ala Trp Pro Gly Gln Arg Glu Arg Ala Thr Ala

1070 1075 1080

Ile Leu Leu Ala Cys Arg His Leu Pro Leu Ser Phe Leu Ser Ser

1085 1090 1095

Pro Gly Gln Arg Ala Val Leu Leu Ala Glu Ala Ala Arg Thr Leu

1100 1105 1110

Glu Lys Val Gly Asp Arg Arg Ser Cys Asn Asp Cys Gln Gln Met

1115 1120 1125

Ile Val Lys Leu Gly Gly Gly Thr Ala Ile Ala Ala Ser

1130 1135 1140

<210> 2

<211> 4249

<212> DNA

<213> homo sapience

<400> 2

```
ccgtcggtga ggcggtgccg ggcgggggtt gtcgggtgtc atgggcggtg gcgacggcac    60
cgcccccgcg tctccctgag cgggacggca gggggggctt ctgcgctgag ccgggcgatg   120
gacgacagcg gcgagctggg tggtctggag accatggaga ccctcacgga gctgggcgac   180
gagctgaccc tgggagacat cgacgagatg ctgcaatttg tcagtaatca agtgggagag   240
ttccctgact tgttttcaga acagctgtgt agctcctttc ctggcagtgg tggtagtggt   300
agcagcagcg gcagcagtgg cagcagcagc agcagcagca atggcagggg cagcagcagc   360
```

ggagctgtgg acccttcagt gcaacggtca ttcacccagg tcacattacc ttccttctct 420
ccctcggcgg cctccccaca ggctccaact ctgcaagtca aggtttctcc cacctcagtt 480
cccaccacac ccagggcaac tcctattctt cagccccgcc cccagcccca gcctcaacct 540
caaactcagc tgcaacaaca gacggtaatg atcacgccaa cattcagcac cactccgcag 600
acgaggatca tccagcagcc tttgatatac cagaatgcag ctactagctt tcaagtcctt 660
cagcctcaag tccaaagcct ggtgacatcc tcccaggtac agccggtcac cattcagcag 720
caggtgcaga cagtacaggc ccagcgggtg ctgacacaaa cggccaatgg cacgctgcag 780
acccttgccc cggctacggt gcagacagtt gctgcgccac aggtgcagca ggtcccggtc 840
ctggtccagc ctcagatcat caagacagat tcccttgttt tgaccacact gaagacagat 900
ggcagccctg ttatggctgc ggtccagaac ccggccctca ccgccctcac cacccctatc 960
cagacggctg cccttcaagt accaaccctg gtgggcagca gtgggaccat tctgaccaca 1020
atgcctgtaa tgatggggca agagaaagtg cccattaagc aggtacctgg gggagtcaag 1080
cagcttgagc cccccaaaga aggagaaagg cggacaaccc ataatatcat tgagaaacga 1140
tatcgctcct ccatcaatga caaaatcatc gaattgaaag acctggtcat ggggacagac 1200
gccaagatgc acaagtctgg cgttctgagg aaggccattg attacatcaa atacttgcag 1260
caggtcaatc ataaactgcg ccaggagaac atggtgctga agctggcaaa tcaaaagaac 1320
aagcttctaa agggcatcga cctaggcagt ctggtggaca atgaggtgga cctgaagatc 1380
gaggacttta atcagaatgt ccttctgatg tccccccag cctctgactc agggtcccag 1440
gctggcttct ctccctactc cattgactct gagccaggaa gccctctatt ggatgatgca 1500
aaggtcaaag atgagccaga ctctcctcct gtggcgctgg gcatggtaga ccgctcacgg 1560
attcttctgt gtgtcctcac cttcctgtgc ctctccttta accccctgac ttccctgctg 1620
cagtggggag gggcccacga ctctgaccag cacccacact caggctctgg ccgcagtgtc 1680
ctgtcattcg agtcaggttc tgggggctgg tttgactgga tgatgcctac tcttctctta 1740
tggctggtaa atggtgtgat tgtcctgagc gtctttgtga agctgctggt tcatggggag 1800
ccagtgatcc ggccacactc gcgctcctcg gtcaccttct ggaggcaccg gaaacaggca 1860
gatctggatc tcgccagagg agattttgca gctgctgccg ccaacctaca aacctgcctg 1920
gcagttttgg gccgggcact gcccacctcc cgcctggacc tggcctgcag cctctcctgg 1980
aacgtgatcc gctacagcct gcagaagcta cgcctggtgc gctggctgct caagaaagtc 2040
ttccagtgcc ggcgggccac gccagccact gaggcaggct ttgaagacga agctaagacc 2100

```
agcgcccggg atgcggctct ggcctatcac cggctgcacc agctgcacat cacagggaag   2160
cttcctgcag gatccgcctg ttccgatgta cacatggcgt tgtgtgccgt gaacctggct   2220
gaatgtgcag aggagaagat cccaccgagc acactggttg agatccatct gactgctgcc   2280
atggggctca agacccggtg tggaggcaag ctgggcttcc tggccagcta cttcctcagc   2340
cgagcccaga gcctgtgtgg ccccgagcac agtgctgttc ctgactccct gcgctggctc   2400
tgccaccccc tgggccagaa gtttttcatg gagcggagct ggtctgtgaa gtcagctgcc   2460
aaggagagtc tatactgtgc ccagaggaac ccagctgacc ccattgcgca ggtccaccag   2520
gccttctgca agaacctgct ggagcgagct atagagtcct tggtgaaacc tcaggccaag   2580
aagaaggctg gagaccagga agaagagagc tgtgaattct ccagtgctct ggagtacttg   2640
aaattacttc attcttttgt ggactctgtg ggggttatga gcccccccact ctccaggagc   2700
tccgtgctca agtccgccct gggtccagac atcatctgtc ggtggtggac gtctgcaatc   2760
actgtggcca tcagctggct ccagggagac gatgcagctg tgcgctctca ttttaccaaa   2820
gtggaacgca tccccaaggc cctggaagtg acagagagcc ccctggtgaa ggccatcttc   2880
catgcctgca gagccatgca tgcctcactc cctgggaaag cagatgggca gcagagttcc   2940
ttctgccatt gcgagagggc cagtggccac ctatggagca gcctcaacgt cagtgggggc   3000
acctctgacc ctgccctcaa ccacgtggtc cagctgctca cctgtgacct gctactgtcg   3060
ctacggacag cgctctggca aaaacaggcc agtgccagcc aggctgtggg ggagacctac   3120
cacgcgtcag gcgctgaact ggcgggcttc caacgggacc tgggcagcct gcgcaggctg   3180
gcacacagct tccgcccagc ataccgcaag gtgttcctgc atgaagccac cgtgcgcctg   3240
atggcaggag gcagccccac ccgcacccac cagctgctgg aacacagcct gcggcggcgc   3300
accacgcaga gcaccaagca cggagaggtg gatgcctggc ccggccagcg agagcgggcc   3360
accgccatcc tgctggcctg ccgccacctg cccctctcct tcctctcctc cccgggccag   3420
cgggcagtgc tgctggccga agctgcccgc accctggaga aggtgggcga ccggcgctcc   3480
tgcaacgact gccagcagat gattgttaag ctgggtggtg gcactgccat tgccgcctcc   3540
tgaccaccag gctcagccca cccctccacc tctctctcga tttctctctc tccccctcag   3600
catcttcccg ctgagagtgg tggggaagag ccttgtcttc ttagctgtca cctgccgagg   3660
cttctgggcc actcaggcca gtgcacccct gggcagagcc ccttaaagct gctgtcacta   3720
gatgcccatg gtccagggcc tggtgggcgt gagaggatag gtggcagggc agaaactggg   3780
cagccctgac ttgatagcag cagggggagc tcccaagctg ccaagcccct gcctccagcc   3840
```

ttcctgagtt tctctctcct gaaccctact ctctcctttt tgcttcctca gtttttatca 3900
ggctttctct gggggacagc agtctctgag caccagggag cagttgccct caggcctgtg 3960
cccagcatgc cctccccttt ttatacgaat gttttctacc agtgtgcttg ggtttgccat 4020
gatgcgaggc tgagttgctg tagcgtcttg attctctccc tgggtctgcg ttccctcccc 4080
tgggcctgac tgagcctgct cattgttttt ccctttatta cacaggacag ccaggggagg 4140
agggggccc agccctggga ggctggtggg aggcaggggg caggcctgcg gatgcatgaa 4200
ataatgttgg cattattttt taattttta aaaaataaat ggtatctta 4249

<210> 3
<211> 26
<212> DNA
<213> primer

<400> 3
tctcaactcc ttgaaaatag ctacct 26

<210> 4
<211> 26
<212> DNA
<213> primer

<400> 4
ggtctatgat tgtagctggt cactta 26

<210> 5
<211> 23

<212> DNA

<213> primer

<400> 5

agcttctaaa gggcatcgac cta 23

<210> 6

<211> 28

<212> DNA

<213> primer

<400> 6

tagaggacac ctagtcagac aaaatgat 28

<210> 7

<211> 24

<212> DNA

<213> primer

<400> 7

ccaactctgc aagtcaaggt ttct 24

<210> 8

<211> 23

<212> DNA

<213> primer

<400> 8
gcgtgatcat taccgtctgt tgt 23

<210> 9
<211> 20
<212> DNA
<213> primer

<400> 9
aggaaacttc gctcacacct 20

<210> 10
<211> 20
<212> DNA
<213> primer

<400> 10
gccatgtatc tgttttggcc 20

<210> 11
<211> 29
<212> DNA
<213> primer

<400> 11

cagcagccca gcagaggttt tctacaatc 29

<210> 12
<211> 23
<212> DNA
<213> primer

<400> 12
tggctcacag ttcttcataa cca 23

<210> 13
<211> 23
<212> DNA
<213> primer

<400> 13
atgacatcct tcagtggctt gtc 23

<210> 14
<211> 26
<212> DNA
<213> primer

<400> 14
tccatgccct ctagaacttt gccgaa 26

## Description

## Technical Field

This invention relates to a non-human transgenic animal as a type 2 diabetes model, a method for screening therapeutic agents for diabetes using the model animal, and a method for preparing the type 2 diabetes model animal.

## Background Art

Diabetes is called as a contemporary national disease as a central core of lifestyle-related diseases, and developments of preventive and therapeutic methods thereof are problems to be urgently solved. While the mechanism of onset of the disease has not been elucidated yet, it is considered that two disease conditions of deficiency of insulin as a hormone for lowering the blood glucose level (insufficiency of insulin secretion) and impairment of the insulin action (insulin resistance) are intermingled. Usually, diabetes is classified into: (1) type 1 requiring continuous replenishment of insulin since β-cells of the pancreas for producing insulin are destroyed; (2) type 2 in which secretion of insulin is deficient or action of insulin is deteriorated; (3) other diabetes by specific causes; and (4) gestational diabetes and the like.

Type 1 diabetes is one of autoimmune diseases, and is clinically called as insulin dependent diabetes. The β-cells of the pancreas that secrete insulin are destroyed by being attacked by the autoimmune system. Since insulin is a hormone for allowing blood glucose to be absorbed by cells to decrease the blood glucose level, glucose in the blood increases while glucose in the cell becomes deficient when the amount of secretion of insulin decreases. The cells cannot maintain their life activities when such glucose deficient state persists, and various impairments of organs, loss of sight and necrosis of foots are caused. The model mouse of type 1 diabetes is known in the art, and studies on the therapy of type 1 diabetes have been advanced using a mouse model (for example, see Science, 2003, Nov. 14; 302 (5648):p1223-7).

Type 2 diabetes is often called as insulin independent diabetes from the clinical point of view, and the disease is developed by insufficient insulin secretion from the pancreatic β-cells and by insulin resistance. Whether insufficient insulin secretion or insulin resistance is strongly concerned with type 2 diabetes is different depending on respective cases or the process of the case, both types of causes are often intermingled. While insulin is instantaneously secreted in response to the start of increase of the blood glucose level by absorbing glucose after the meal in normal person, this response is lacking in the insulin secretion deficient case, and insulin is secreted by being retarded from the increase of blood glucose level. Insulin resistance refers to a state in which the action of insulin is impaired by various reasons, although insulin is secreted. The immunological mechanism of type 1 diabetes is clear, and a model mouse expressing the disease condition has been already reported as described above. However, since the cause of type 2 diabetes is complicated, mouse model capable of sufficiently describing the human disease condition has not been reported yet.

On the other hand, SREBP (Sterol Regulatory Element Binding Protein) family belongs to s transcription factor family for controlling sterol synthesis, and binds to a specific sequence SRE common to a cholesterol synthesis enzyme group and LDL receptor gene promoter region for controlling expression of transcription. SREBP is known to include two subfamilies of SREBP-1 (SREBP-1a and SREBP-1c) and SREBP-2. Former studies have revealed that SREBP-1c is responsible for the synthesis of fatty acids and neutral lipids (triglycerides), and SREBP-2 is responsible for the synthesis of cholesterol (J. Clin. Invest. 1996, 98, 1575-1584; J. Clin. Invest. 1997, 99: 846-854; J. Clin. Invest. 1998, 101:2331-2339; Cell, Review, 1997; 331-40). However, there have been no reports on the relation between SREBP-2 and diabetes, particularly between SREBP-2 and type 2 diabetes.

Japanese Patent Application National Publication No. 2003-501102 discloses an animal model of a genetically modified fly or threadworm for expression or abnormal expression of SREBP protein. However, the experimental animal described in this patent publication (fly or threadworm) is developed for use in the study on lipid metabolism. This patent publication does not suggest the relation between SREBP protein and diabetes at all.

## Disclosure of Invention

The number of diabetes patients in this country is estimated to be from 6 to 7 million, and the number of latent patients is supposed to be approximately the same. However, no mechanism of onset of the disease has been elucidated, and no effective therapeutic agents have been developed today. In particular, since the proportion of type 2 diabetes is as large as from 90 to 97%, developments of therapeutic methods and therapeutic agents have been strongly desired.

The inventors of the invention have developed a mouse capable of specifically and forcibly expressing SREBP-2 in the pancreatic β-cells that are insulin-secreting cells by introducing a DNA encoding the human active SREBP-2 protein in mouse. Although an expected change has been variation of metabolism of cholesterol (activation of synthesis) in the pancreatic β-cells, surprisingly insulin secretion is remarkably reduced with an increase of the blood glucose level in addition to abnormal metabolism of cholesterol, and a symptom resembling to type 2 diabetes is found to be manifested.

Such disease condition is different from the action mechanism according to conventional theories of lipid toxicology that secretion of insulin decreases by a fatty acid excess state and accumulation of neutral lipids (triglycerides) in the cell. In other words, variation of cholesterol metabolism or SREBP-2 itself may be a cause of type 2 diabetes. This discovery is quite epoch-making considering that no reports have been found on the relation between the decrease of insulin secretion and diabetes related to the metabolism of cholesterol.

The invention completed based on the above-mentioned discoveries provides a non-human transgenic animal as a model of type 2 diabetes, a method for screening a therapeutic agent of diabetes using the model animal, and a method for preparing the model animal for type 2 diabetes as follows:

(1) a non-human transgenic animal as a model of type 2 diabetes manifesting a symptom of type 2 diabetes by excessive expression of the active SREBP-2 protein in pancreatic β-cells by introducing a recombinant DNA in which a DNA encoding the active SREBP-2 protein is disposed under the control of a promoter;
(2) the transgenic animal according to (1), wherein the symptom of type 2 diabetes includes abnormal cholesterol metabolism and impaired insulin secretion in the pancreatic β-cells;
(3) the transgenic animal according to (1), wherein the DNA encoding the active SREBP-2 protein is a human active SREBP-2 cDNA;
(4) the transgenic animal according to (1) or (2), wherein the promoter is a promoter of rat insulin I gene;
(5) the transgenic animal according to any one of (1) to (4), wherein a gene marker is further introduced into the recombinant DNA;
(6) the transgenic animal according to (5), wherein the gene marker is a green fluorescent protein;
(7) the transgenic animal according to any one of (1) to (6), wherein the animal is mouse, rat or rabbit;
(8) the transgenic animal according to (7), wherein the animal is mouse;
(9) a method for screening a therapeutic agent for type 2 diabetes using the transgenic animal according to any one of (1) to (8);
(10) the method for screening a therapeutic agent for type 2 diabetes according to (9) comprising the step of observing the change of the symptom of type 2 diabetes after administering a test compound to the transgenic animal; and
(11) a method for preparing a transgenic animal manifesting the symptom of type 2 diabetes comprising the steps of: constructing a recombinant DNA in which a DNA encoding a human active SREBP-2 protein is disposed under the control of a promoter; introducing the recombinant DNA and a gene marker into fertilized ovum of a non-human animal; transplanting the fertilized ovum into a pseudo-pregnant non-human mammal to breed the mammal; and selecting infants having the recombinant DNA from the delivered infants using an expression product of the gene marker as an index.

The transgenic animal of the invention as a novel model animal of type 2 diabetes is useful as a tool for studying the mechanism of onset of diabetes and for screening a therapeutic agent of diabetes. While many other existing model animals of diabetes are required to have high lipid diet as an essential condition of onset of diabetes or the animals are evidently obese, the transgenic mouse according to the preferred aspect of the invention spontaneously develops diabetes without being obese, and the disease condition resembles to the disease condition of type 2 diabetes dominant in Japanese. Accordingly, the transgenic mouse according to the invention is useful as a model mouse of type 2 diabetes manifesting above-mentioned disease conditions.

## Brief Description of the Drawings

Fig. 1 shows a construction of the SREBP-2 gene expression vector.
Fig. 2 is a graph showing the fasting blood glucose level and fasting insulin level in the SREBP-2 transgenic mouse.
Fig. 3 is a graph showing the changes of the blood glucose level and of the blood insulin level in an intravenous glucose loading test.
Fig. 4 shows insulin secretion ability by glucose stimulation of isolated Langerhans' islet.
Fig. 5 shows a comparison of the amounts of expression between transgenic human SREBP-2 and HMGCoA synthetase.

## Best Mode for Carrying Out the Invention

### 1. Non-human transgenic animal as a model animal of type 2 diabetes

The invention provides a novel model animal type 2 diabetes that decreases insulin secretion ability by allowing pancreatic β-cells to excessively express a cholesterol synthesis control transcription factor SREBP-2. Such a model animal is useful for elucidating the mechanism of onset of type 2 diabetes and for developing therapeutic agents.

Specifically, a first aspect of the invention provides a non-human transgenic animal as a model of type 2 diabetes, wherein an introduced recombinant DNA having a DNA encoding an active SREBP-2 protein is disposed under the control of a promoter, and the animal manifests symptoms of type 2 diabetes by forced excessive expression of SREBP-2 in the pancreatic β-cells.

The "SREBP-2 protein" as used in the present specification is one of SREBP subfamily as a sterol regulatory element binding protein, and a known membrane-bound protein related to regulation of cholesterol synthesis. The SREBP-2 gene and SREBP-2 protein are described, for example, in Proc. Natl. Acad. Sci. USA, 1993, Dec. 15; 90(24): 11603-7. In particular, the amino acid sequence of the human SREBP-2 protein is deposited with NCBI and given Accession No. AAA50746, and is represented by SEQ ID No. 1. The base sequence of the human SREBP-2 cDNA is deposited with GenBank and is given Accession No. U02031, and is expressed by SEQ ID No. 2.

Endogenous expression of SREBP-2 is widely distributed in almost all tissues, although the extent of expression is a little different in respective tissues. However, SREBP-2 is present as a membrane-bound protein that is quite unique as a transcription factor, and cannot directly exhibit its activity as the transcription factor. The active part is cleaved only when cholesterol requirement of the cell is enhanced, transferred into nuclei, and the cell supplies cholesterol by enhancing expression of a group of genes of a cholesterol synthesis system. On the contrary, the transcriptional activity of SREBP-2 is not exhibited without cleaving the active part when cholesterol is abundant. This system permits the amount of cholesterol in the cell to be appropriately controlled.

Since the control mechanism of cholesterol caused by the SERBP-2 is a system usually working in all the cells, the same mechanism is supposed to be valid in normal β-cells. Since only the portion transferred into the nucleus, or active SREBP-2, is forcibly and excessively expressed in the transgenic animal of the invention, above-mentioned feedback is not valid and cholesterol synthesis is forcibly in an enhanced state. In other words, the activity is maintained to be specific to the β-cell in the transgenic animal of the invention to consequently manifest the symptom of type 2 diabetes. Examples of symptoms of type 2 diabetes include abnormal metabolism (excess synthesis) of cholesterol and impairment of insulin secretion in the pancreatic β-cell as well as hyperglycemia, positive urine sugar and no occurrence of obesity. These symptoms can be judged by a skilled person in the art by known methods. For example, hyperglycemia and impaired insulin secretion can be judged by investigating the tendency of hyperglycemia and decline of insulin secretion in the glucose load test. Whether these symptoms are manifested or not may be judged by those skilled in the art. For example, excess synthesis of cholesterol may be judged by an increase of the amount of cholesterol of 10% or more, more definitely 20% or more, and further definitely 30% or more in the model animal group as compared with a normal animal group, when the Langerhans' islet is isolated from the pancreas of mouse and lipids are extracted to measure the content of cholesterol. Impaired insulin secretion can be judged by a decrease of the amount insulin of 20% or more, more definitely 30% or more, in the culture medium of the model animal group as compared with the normal animal group, when the Langerhans' islet is isolated from the pancreas of mouse and the amount of insulin secretion is measured in a high glucose medium. Alternately, impaired insulin secretion due to decline of the insulin secretion ability in a living animal may be judged for each animal by a statistically significant decrease of the insulin concentration of the model animal group as compared with the normal animal group, when a mouse is subjected to the sugar load test, or the mouse is loaded with glucose, the blood of the mouse is sampled with time, and the blood insulin concentration is measured. Hyperglycemia may be judged by a statistically significant increase of the blood glucose level of the model animal group as compared with the normal animal group in the glucose load test or repeated sampling of the blood. Since diagnostic criteria for human cannot be simply applied for the diagnosis of diabetes of the animal, the animal may be diagnosed as diabetes when the blood glucose level is constantly high and urine sugar is positive. The decrease of insulin secretion ability also strongly supports the diagnosis of diabetes when the animal develops diabetes of declined insulin secretion type.

The "active SREBP-2 protein" as used in the present specification refers to an active part of the cleaved SREBP-2 protein as mentioned above or mutated proteins thereof. In more detail, the "active SREBP-2 protein" is a protein having amino acid residues of 1st to 460th amino acid sequences represented by SEQ ID No. 1 (human active type) or mutated proteins thereof, and has an activity as a transcription factor against cholesterol synthesis-related genes. While the active SREBP-2 protein of the invention may be derived from any species, it is preferably a protein derived from human. While the "mutated protein" is not particularly restricted so long as it has a function as a transcription factor against the cholesterol synthesis-related genes, the protein is a mutated protein comprising, for example, deletion, substitution, insertion and/or addition to the amino acid sequence of the human protein. While the mutation site and number of the amino acids are not particularly restricted so long as the mutated protein maintains the transcription activity, the number of mutation is usually at 1 to 30 amino acid residues, preferably at 1 to 10 amino acid residues, and more preferably at 1 to several amino acid residues (for example 6 amino acid residues).

While the animal species as the object of the invention is not particularly restricted, examples of the preferable animal include mouse, rat, rabbit, goat and cattle. Mouse is most preferable among them since preparation of the mouse line is easy.

## 2. Preparation of transgenic animal

The invention also provides a method for preparing the transgenic animal. Specifically, the method for preparing the transgenic animal of the invention comprises the steps of: (1) constructing a recombinant DNA in which a DNA encoding a human active SREBP-2 protein is disposed under the control of a promoter; (2) introducing the recombinant DNA and a gene marker into a fertilized ovum of a non-human animal; (3) transplanting the fertilized ovum obtained into a pseudo-pregnant non-human animal; (4) breeding the animal; and (5) selecting infants having the recombinant DNA in the chromosome from delivered infants using an expression product of the gene marker as an index, to select a line in which the protein derived from the recombinant DNA is stably expressed in a desired tissue after establishing the line by breeding.

The method for preparing the transgenic animal (for example mouse) is well known, and is described in detail in (1) Method in Enzymology, Vol. 225, "Guide to Techniques in Mouse Development", edited by Paul M. Wassarman, Melvin L. DePamphilis, Academic Press, Inc.; (2) "Up-to-Date Technology of Gene Targetting", Yodo-Sha Co.; and (3) Gordon, J. W., 1993, "Guide to Techniques in Mouse Development", (Wassarman, P. M. and DePamphilis, M. L., Eds.), Academic Press, San Diego. PCR, production of primers, production of genome DNAs, cloning and enzyme processing methods can be performed by conventional methods well known to those skilled in the art (see "Molecular Cloning, A Laboratory Manual", Third Edition, Cold Spring Harbor Press, Cold Spring Harbor Laboratory, Cold Spring Harbor, New York, 2001).

Since the animal preferably used in the invention is the mouse as described above, the method for preparing the non-human transgenic animal of the invention is described with reference to the method for preparing the transgenic mouse.

For preparing the transgenic mouse of the invention, the recombinant DNA (or a DNA construct) is constructed so that the active SREBP-2 gene used in the invention is disposed under the control of a promoter. The DNA encoding the active SREBP-2 protein used in the invention may be produced by many methods including DNA synthesis, cloning of cDNA, cloning of genomes, polymerase chain reaction (PCR) and a combination of these technologies (for example, see "Molecular Cloning, A Laboratory Manual", Third Edition, Cold Spring Harbor Press, Cold Spring Harbor Laboratory, Cold Spring Harbor, New York, 2001).

The DNA encoding the active SREBP-2 protein used in the invention can be constructed by conventional gene recombination techniques. For example, the DNA is obtained by screening a cDNA library by a PCR method or hybridization method using a primer or a probe designed and synthesized based on information on known sequences of amino acids and bases. The SREBP-2 protein can be expressed in an appropriate host by forming a DNA construct or an expression vector produced by linking the desired DNA thus obtained at the downstream of an appropriate promoter. The DNA encoding the active SREBP-2 protein used in the invention is preferably a human active SREBP-2 cDNA.

Examples of the vector used in the invention include plasmids, cosmids and viruses (including phages). These vectors can be also designed and constructed using conventional gene recombination technologies. These vectors are usually linked in the direction from a 5'-end to a 3'-end so that the promoter and human active SREBP-2 gene can be expressed. While the promoter is not particularly restricted so long as the SREBP-2 gene is able to be specifically expressed to be specific to the pancreatic β-cell, examples of the promoter include the promoter of rat insulin I gene promoter (Alpert, S., Hanahan D, Teitelman G. Cell, 1988, Apr., 22;53(2): 295-308) and amylin promoter. The promoter of rat insulin I gene is particularly preferable in the invention. An enhancer, a silencer and a poly A addition signal may be integrated into the expression vector so that the desired gene is properly expressed.

It is preferable that the gene marker is introduced into the vector together with the DNA used in the invention. Examples of such gene marker include green fluorescent proteins (such as GFP and EGFP), luciferase, β-galactosidase and chloramphenicol acetyltransferase. When the gene marker is introduced into the expression vector together with the DNA, introduction of the recombinant DNA and gene marker and expression thereof may be efficiently confirmed by using an expression product of the gene marker expressed in an infant mouse as an index.

While examples of the method for introducing the recombinant DNA thus obtained into the fertilized ovum include a microinjection method, a retrovirus vector method or a method using embryonic stem cells, the microinjection method is usually used. The fertilized ovum cultivation method, microinjection method and transplantation method of the fertilized ovum are well known by those skilled to the art.

Subsequently, the fertilized ovum to which the recombinant DNA has been introduced by microinjection is transplanted to the uterine tube of the pseudo-pregnant mouse. The mouse is bred, and the transgenic mouse of the invention can be obtained by selecting an infant mouse having the recombinant DNA from the delivered mice. Selection of the infant mouse having the recombinant DNA from the delivered infant mice can be confirmed by discriminating SREBP-2 produced by introducing the recombinant DNA from the endogenous SREBP-2 by PCR using a specific primer having the base sequence of the human SREBP-2 gene and a part of other base sequences necessary for expression using genome DNA extracted from the tail of the mouse. Otherwise, introduction of the recombinant DNA and gene marker may be confirmed using an expression product of the gene marker as an index, when the gene marker is introduced into an expression vector.

For judging whether the transgenic mouse of the invention can be used as a model mouse of type 2 diabetes or not, expression of the introduced gene in the pancreatic β-cell in the mouse is confirmed at first, and abnormal metabolism of cholesterol in the β-cell as a direct effect of gene introduction is investigated. Finally, impaired insulin secretion in pancreatic β-cell as well as phenotype of diabetes such as hyperglycemia, polyuria and positive urine sugar is confirmed.

**3. Screening method**

The transgenic mouse prepared by the inventors of the invention can be concluded to be the model mouse of type 2 diabetes from the view point of clinical finding, clinical test and pathology as confirmed in the examples hereinafter. Accordingly, the transgenic mouse of the invention as the animal model of type 2 diabetes is useful as a screening tool for elucidating onset mechanisms of type 2 diabetes and for developing therapeutic methods and therapeutic agents thereof.

Another aspect of this invention provides a screening method of the therapeutic agent of type 2 diabetes comprising observing the change of symptoms of diabetes after administering a test compound to the transgenic animal. Specifically, the method for confirming the test compound to be effective or not as a therapeutic agent of type 2 diabetes comprises: grouping the transgenic mouse of the invention into test mouse groups and non-transgenic littermates into reference mouse groups; administering the test compound to the test mouse group; sampling the urine and blood from the test mouse group and reference mouse group to measure the content of glucose in the urine and blood glucose level; and comparing investigation these measured values.

**Examples**

While the invention is described hereinafter with reference to examples and experimental examples, the invention is by no means restricted to these examples.

**(1) Production of SREBP-2 gene expression vector under the control of insulin promoter**

Standard methods were used for molecular biology methods (see "Molecular Cloning, A Laboratory Manual", Third Edition, Cold Spring Harbor Press, Cold Spring Harbor Laboratory, Cold Spring Harbor, New York, 2001). For producing DNAs for microinjection necessary for preparing the transgenic mouse, SREBP-2 gene expression vectors were produced under the control of an insulin promoter. The construction thereof is shown in Fig. 1.

**(Promoter)**

Rat insulin promoter I (Alpert, S., Hanahan, D. and Teitelman, G., Cell, 1988, Apr., 22; 53(2): 295-308) that has been successful for expression in the past Tg mouse was used as an expression promoter for specific expression of the introduced gene in the β-cell of the animal. The portion from -715 bp to +31 bp was obtained by PCR using the primers described below using a rat genome DNA as a template:

5'-primer: 5'-TCTCAACTCCTTGAAAATAGCTACCT-f-3' (SEQ ID No. 3)
3'-primer: -GGTCTATGATTGTAGCTGGTCACTTA-r-3'(SEQ ID No. 4)
Platinum Pfx DNA polymerase (manufactured by Invitrogen Co.) was used as a DNA polymerase.

**(Expression cDNA)**

Human SREBP-2 cDNA expression vector pCMVh SREBP-2 (an expression vector of cDNA containing a transfer active portion (1 to 460 amino acid residues) of human SREBP-2; Amemiya-Kudo M, Shimano H, Hasty A H, Yahagi N, Yoshikawa T, Matsuzaka T, Okazaki H, Tamura Y, Iizuka Y, Ohashi K, Osuga J, Harada K, Gotoda T, Sato R, Kimura S, Ishibashi S, and Yamada N, J. Lipid Res. 2002, 43(8), 1220-35) was used as the cDNA of human SREBP (sterol regulatory element-binding protein).

Poly A signal of human growth hormone in the expression vector was used for the poly A signal for terminating transcription. The DNA was fused in plasmid blue script II SK(+/-) (manufactured by Stragene) as shown in Fig. 1 to construct an expression vector. The base sequence of the DNA portion obtained by PCR was confirmed by DNA sequencing. The function of the expressed vector was confirmed by Luciferase reporter assay of the transcription activity of SREBP-2 transfected into a cell line of the β-cell and by western blotting of the expressed protein.

**(2) Preparation of microinjection DNA**

The plasmid DNA was purified in large scale using a plasmid purification kit (manufactured by Quiagen Co.), and the purified DNA was cleaved with restriction enzymes NotI and XhoI. The injected DNA portion shown in Fig. 1 was separated by agarose electrophoresis, and was purified using QIAEX II Gel Extraction Kit (manufactured by Quiagen Co.).

**(3) Preparation of transgenic mouse**

The transgenic mouse was prepared according to a published report (Method in Enzymology, Vol. 225, "Guide Techniques in Mouse Development", edited by Paul M. Wassarman, Melvin L. DePamphilis, Academic Press, Inc.). An outline of the preparation method comprises: preparing the expression gene obtained in (1) in male pronuclei of the fertilized ovum (BDF2) obtained by natural mating between B6SJL/F2 mice; introducing about 1000 copies of the expression gene by a micro-injection method (based on USP No. 4,873,191 by Ohio University); and transplanting the expression vector into the uterine tube in an ICR pseudo-pregnant mouse (0.5 dpc). The survived infant mouse was extracted by Caesarean section or by spontaneous delivery from the pregnant mouse at 19.5 dpc after transplantation to obtain 45 F0 mice.

Introduction of injected gene into the genome DNA of five F0 mice was confirmed by PCR as described hereinafter by extracting the tail DNA from 45 mice obtained. The PCR method was also used for screening of genotypes.

Particularly, the following primer sets specific to the introduced gene portion were used for the reaction under the following conditions using the tail DNA as a template:

Primer:

(hBP-2 side, sense): 5'AGCTTCTAAAGGGCATCGACCTA3' (SEQ ID No. 5)
(hGH side, antisense): 5'TAGAGGACACCTAGTCAGACAAAATGAT3' (SEQ ID No. 6)

**PCR condition:**

$$94°C \times 4\ min. \times 1\ cycle$$

$$94°C \times 1\ min. \times 35\ cycle$$

$$60°C \times 1\ min. \times 35\ cycle$$

$$72°C \times 1\ min. \times 35\ cycle$$

$$72°C \times 4\ min. \times 1\ cycle$$

**(4) Identification of expression line**

As the result, C57 BL6/J mice (purchased from CLEA Co.) were mated with five founder mice in which introduction of the gene into the chromosome DNA was confirmed. The mouse line was established by confirming introduction of the gene into descendants by PCR of the tail DNA as described above.

Langerhans' islet was separated from the pancreas of each descendant mouse line, total RNA was extracted using TRIzol (trade name, manufactured by Invitrogen Co.), and expression of human SREBP-2 was investigated by a real-time PCR method. Line A exhibiting most frequent expression was established, the mouse of the established line was repeatedly mated with C57BL6/J mouse, and backcrossing was repeated so that breeding and genetic background are close to those of C57BL6/J strain.

During the period of mating, the transgenic mouse obtained by genotyping and non-transgenic littermate mouse were subjected to initial analysis. Transgenic mouse line A was stable and showed a positive ratio of about 50% in the littermate according to Mendel's law. It was confirmed that integration of the introduced gene is stably propagated in the chromosome. Expression of human SREBP-2 mRNA was stably confirmed without dispersion among the individuals by measuring the expression using a qualitative real-time PCR (the method is described later, see Fig. 5) from the RNA derived from Langerhans' islet (isolation method is described later), and the transgenic mouse line excessively expressing β-cell specific human SREBP-2 was considered to be established.

Qualitative real-time PCR was performed as follows. RNA of each sample was extracted using TRIzol, and cDNA was synthesized from the RNA using Thermoscript (manufactured by Invitrogen Co.) by quantitative PCR by TaqMan (Applied Biosystems, ABI) (40 cycles of 50°C/2 minutes and 95°C/10 minutes followed by 95°C/15 seconds and 60°C/1 minute) using ABI Prism 7000 PCR instrument (trade name, manufactured by Applied Biosystems Co.). The primers and probes used were as follows.

**Human SREBP-2:**

5'-CCAACTCTGCAAGTCAAGGTTTCT-f-3' (SEQ ID No. 7)
5'-GCGTGATCATTACCGTCTGTTGT-r-3' (SEQ ID No. 8)

**Mouse HMG CoA synthetase:**

5'-AGGAAACTTCGCTCACACCT-f-3' (SEQ ID No. 9)
5'-GCCATGTATCTGTTTTGGCC-r-3' (SEQ ID No. 10)

**Probe:**

CAGCAGCCCAGCAGAGGTTTTCTACAATC (SEQ ID No. 11)

**Cyclophilin:**

5'-TGGCTCACAGTTCTTCATAACCA-f-3' (SEQ ID No. 12)
5'-ATGACATCCTTCAGTGGCTTGTC-r-3' (SEQ ID No. 13)

**Probe:**

5'-TCCATGCCCTCTAGAACTTTGCCGAA-3' (SEQ ID No. 14)

**(5) Phenotype of prepared mouse: confirmation of diabetes**

Positive urine sugar and polyuria were observed in the transgenic mouse line A obtained as described above during breeding with normal diet, and onset of diabetes was suggested. As shown in Fig. 2, the measurement of fasting glucose level (glucose measuring kit, manufactured by Wako Pure Chemical Industries, Ltd.) showed a significantly higher level of 174 mg/dl in the transgenic mouse group as compared with the level of 99 mg/dl in the non-transgenic littermate reference group, and the insulin level was below the measuring sensitivity (ELISA kit, manufactured by Shibayagi Co.). The blood glucose level under daily feeding evidently showed a level of 300 to 500 mg/dl corresponding to diabetes.

In the glucose load test of the transgenic mouse group (glucose was injected into the vein of the tail for glucose load test (1 g/kg, 20% aqueous solution), the blood was sampled from the vein of the orbit with time (before injection and 5, 15 and 30 minutes after injection), and blood glucose level and blood insulin level were measured. The time-dependent pattern showed a diabetes pattern of the impaired insulin secretion type (Fig. 3). The level was significantly higher in the transgenic mouse than in the control at each time point, and the high glucose level after the load of glucose persisted. Increase of the blood insulin level corresponding to elevation of the blood glucose level, which was observed in the reference group, was scarcely observed, and the transgenic mouse group showed evidently impaired secretion of insulin.

When Langerhans' islet was separated for directly confirming impaired secretion of insulin, the size of each islet was reduced to about 50% with abnormal shape of the islet, which showed impairment of Langerhans' islet. When insulin secretion ability of the Langerhans' islet cells during cultivation was measured by making the size of the cells uniform after separation, the amount of insulin secretion against the concentration of glucose in the culture medium showed 50% decrease as shown in Fig. 4, and decline of the insulin secretion ability was observed for respective Langerhans' islets.

Langerhans' islet was isolated as follows. The mouse was slaughtered by dislocation of the cervical spine, and 2.5 ml of a collagenase solution (4 mg/ml, 10 mM HEPES (pH 7.4), 0.5% BSA (KRBH)) was injected into the pancreatic duct after ligating the bile duct. The swelled pancreas was further cultivated at 37°C for 3.5 minutes and washed with KRBH, and isolated Langerhans' islet was collected under a stereoscopic microscope.

It was confirmed in the established human SREBP-2 transgenic mouse using the rat insulin promoter that the mouse developed diabetes due to impaired secretion of insulin. For investigating the amount of expression of each line, as shown in Fig. 5, a part of the same investigation was performed with respect to line B in which the amount of expression of human SREBP-2 is smaller than line A, and it was confirmed that each investigation item concerning diabetes slightly exists in response to the smaller amount of expression of human SREBP-2. In other words, it was confirmed that the mouse exhibits a phenotype depending on the amount of expression of the introduced SREBP-2 gene, and the phenotype of line A was caused by the effect depending on the amount of the expressed SREBP-2 gene, not by the non-specific effect by introducing the gene.

**(6) Disease condition of diabetes of prepared mouse**

The transgenic mouse prepared as described above manifests the disease condition of type 2 diabetes in that secretion response to simulation with glucose rather than basal secretion is impaired, not by depletion of insulin by abolition of the β-cell as seen in type 1 diabetes. The body weight, appearance and the weight of the anatomical adipose tissue of the mouse are almost identical to those of the normal mouse, and no obesity is observed. In other words, the disease condition of this mouse model is evidently different from that of the obese mouse model and insulin resistant mouse model, although these models equally suffers from type 2 diabetes, and the model mouse of this invention serves as the type 2 diabetes model mouse of the non-obese and insulin secretion impaired type that is frequently found in Japanese. As shown in Fig. 5, expression of HMG CoA synthetase as a key enzyme in the cholesterol synthesis system is induced depending on the amount of expression of the introduced gene. Accordingly, a relation between the expression of HMG CoA synthetase and onset of diabetes was conjectured since cholesterol synthesis in the β-cell is supposed to be activated in the mouse of the invention.

The transgenic mouse prepared as described above suffers from diabetes due to a novel mechanism of impaired secretion of insulin, and encompasses new concept of onset of diabetes. Accumulation of neutral lipids in β-cell has been noticed as pathogenesis of impaired secretion of insulin. The mouse of the invention that is anticipated to accumulate cholesterol may be a quite novel diabetes model that suggests a possibility of quite new pathological mechanism of impairment of metabolism of cholesterol and secretion of insulin.

**Industrial Applicability**

The non-human transgenic animal of the invention is useful as a tool for investigating the onset mechanism of type 2 diabetes and as a tool for screening therapeutic agents of type 2 diabetes. The transgenic mouse according to a preferable embodiment of the invention is advantageous for investigations since a half of descendants can be used as diseased mice and remaining half of the descendants can be used as normal reference group after breeding. Other known model animals that exhibit disease conditions resembling to type 2 diabetes have been inconvenient for use since the animals evidently manifests obesity or special loading diets such as high fat diets should be given for a long period of time in order to allow the animals to develop diabetes since insulin resistance is emphasized. The mouse of the invention is a quite convenient model in that the mouse is not obese and develops diabetes by lowered level of insulin secretion since younger generations even by being fed on normal diet. It is also advantageous that severe diabetes is developed by feeding on loading diet.

The screening method according to another embodiment of the invention can be effectively used for screening therapeutic agents of type 2 diabetes abundant in Japanese.

## Claims

1. A non-human transgenic animal as a model of type 2 diabetes manifesting a symptom of type 2 diabetes by excessive expression of the active SREBP-2 protein in pancreatic β-cells by introducing a recombinant DNA in which a DNA encoding the active SREBP-2 protein is disposed under the control of a promoter.

2. The transgenic animal according to Claim 1, wherein the symptom of type 2 diabetes includes abnormal cholesterol metabolism and impaired insulin secretion in the pancreatic β-cells.

3. The transgenic animal according to Claim 1, wherein the DNA encoding the active SREBP-2 protein is a human active SREBP-2 cDNA.

4. The transgenic animal according to Claim 1 or 2, wherein the promoter is a promoter of rat insulin I gene.

5. The transgenic animal according to any one of Claims 1 to 4, wherein a gene marker is further introduced into the recombinant DNA.

6. The transgenic animal according to Claim 5, wherein the gene marker is a green fluorescent protein.

7. The transgenic animal according to any one of claims 1 to 6, wherein the animal is mouse, rat or rabbit.

8. The transgenic animal according to Claim 7, wherein the animal is mouse.

9. A method for screening a therapeutic agent for type 2 diabetes using the transgenic animal according to any one of Claims 1 to 8.

10. The method for screening a therapeutic agent for type 2 diabetes according to Claim 9, comprising the step of observing the change of the symptom of diabetes after administering a test compound to the transgenic animal.

11. A method for preparing a transgenic animal manifesting the symptom of type 2 diabetes comprising the steps of: constructing a recombinant DNA in which a DNA encoding a human active SREBP-2 protein is disposed under the control of a promoter; introducing the recombinant DNA and a gene marker into fertilized ovum of a non-human animal; transplanting the fertilized ovum into a pseudo-pregnant non-human mammal to breed the mammal; and selecting infants having the recombinant DNA from the delivered infants using an expression product of the gene marker as an index.

FIG. 1

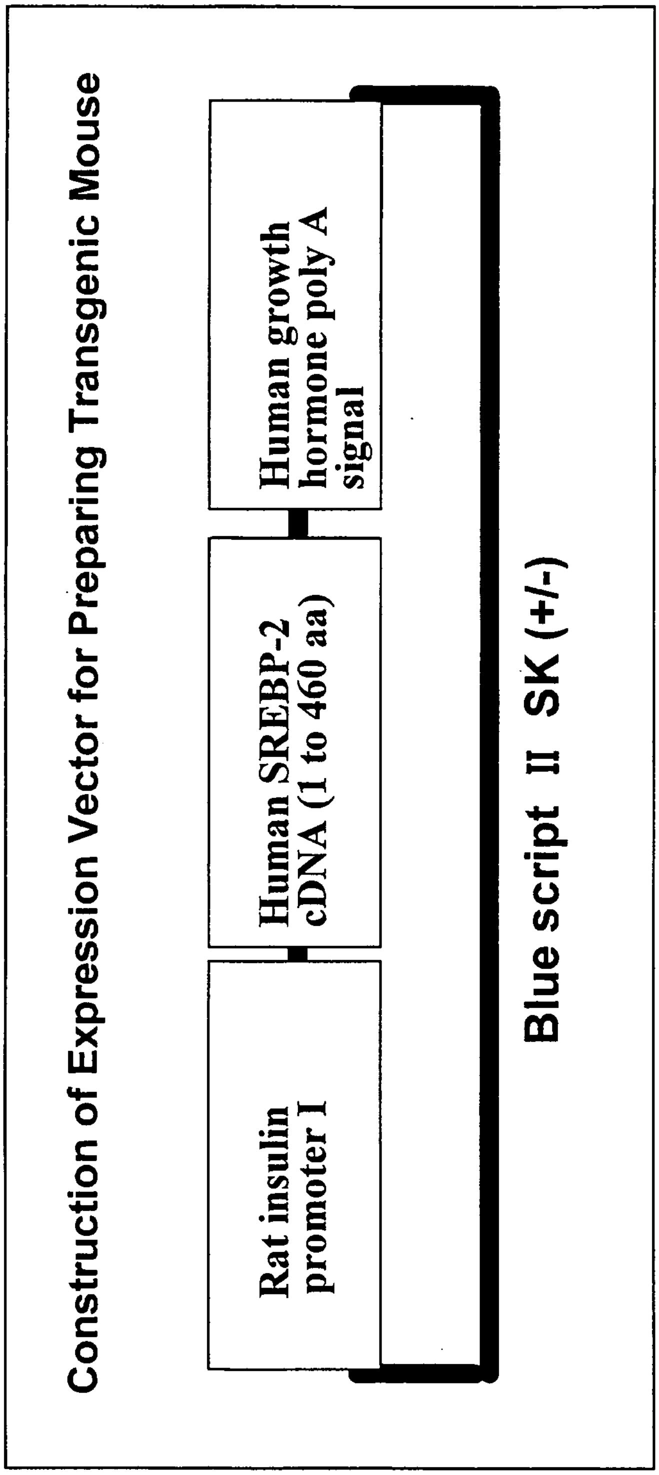
Construction of Expression Vector for Preparing Transgenic Mouse
Rat insulin promoter I
Human SREBP-2 cDNA (1 to 460 aa)
Human growth hormone poly A signal
Blue script II SK (+/-)

# FIG. 2 Fasting Blood Glucose Level and Insulin Level in SREBP-2 Transgenic Mouse

(Fasting Blood Glucose Level)

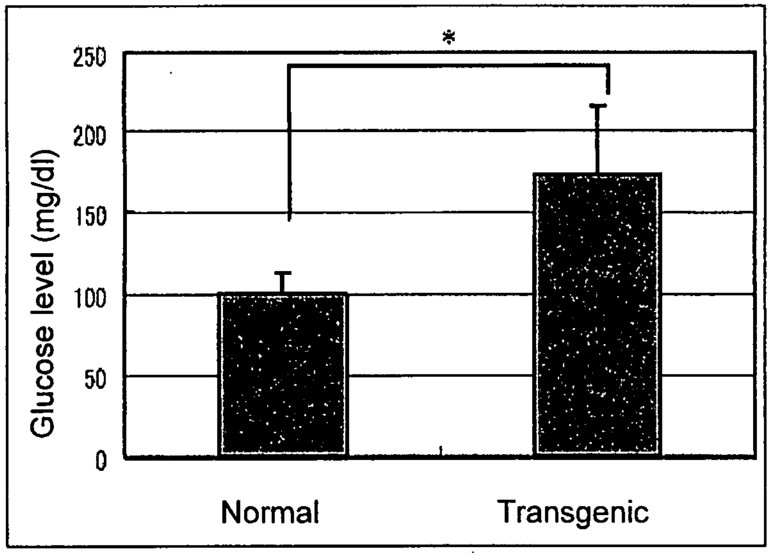


(Insulin Level)

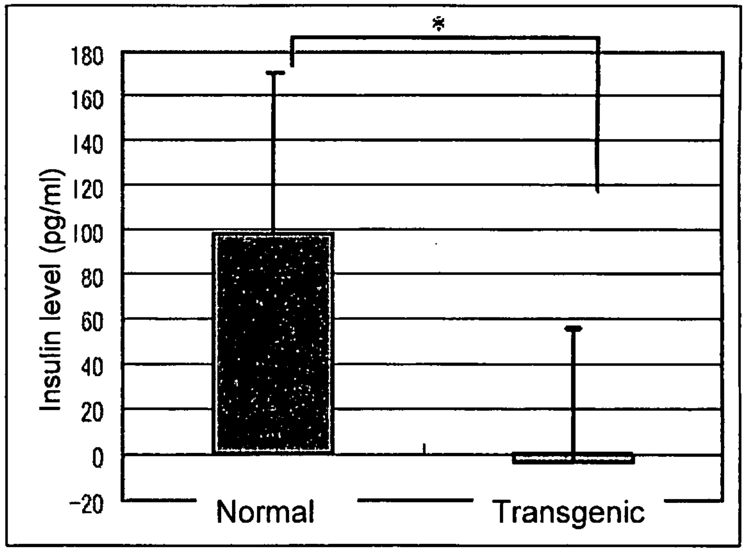


Average value ± SD, n=20 * $p<0.05$ (student t-test)

# FIG. 3 Intravenous Glucose Load Test

(Blood Glucose Level) (Insulin Level)

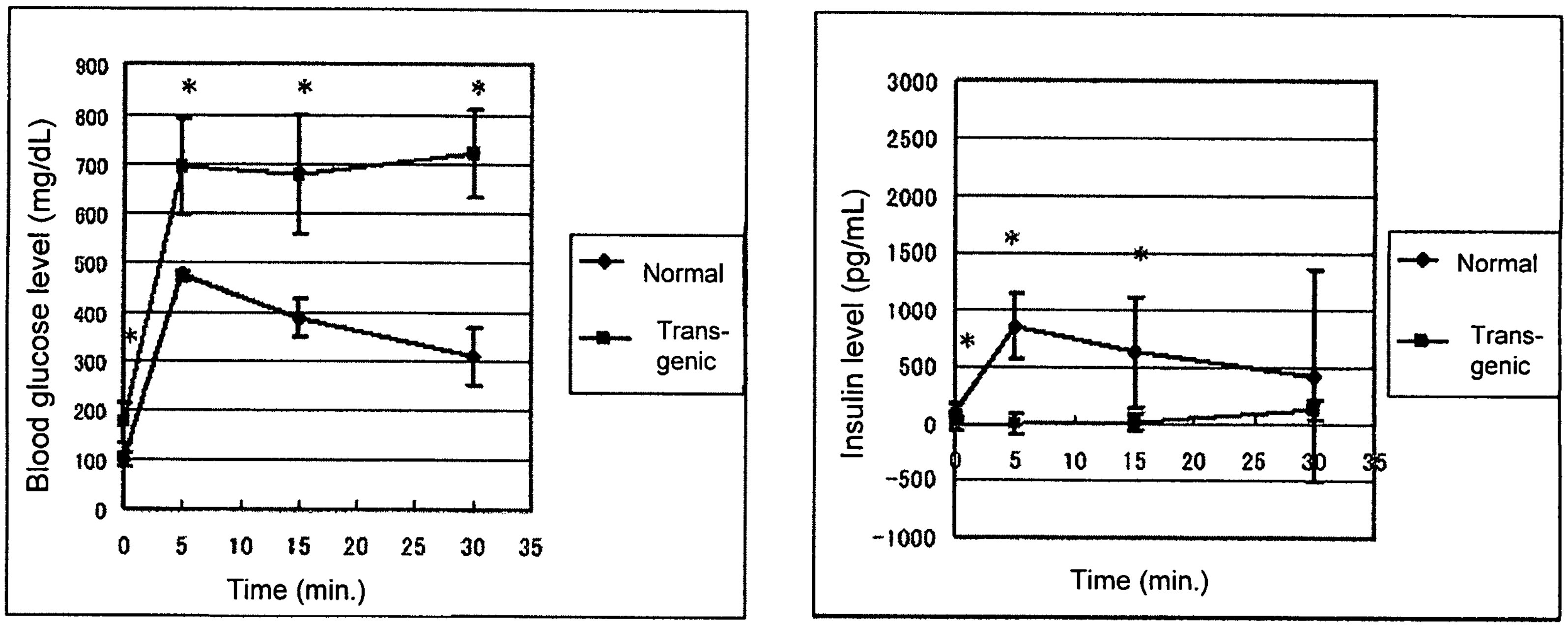


Average value ± SD, n=5 * $p<0.05$ (student t-test)

FIG. 4 Insulin Secretion Ability from Isolated Langerhans' islet by Glucose Stimulation

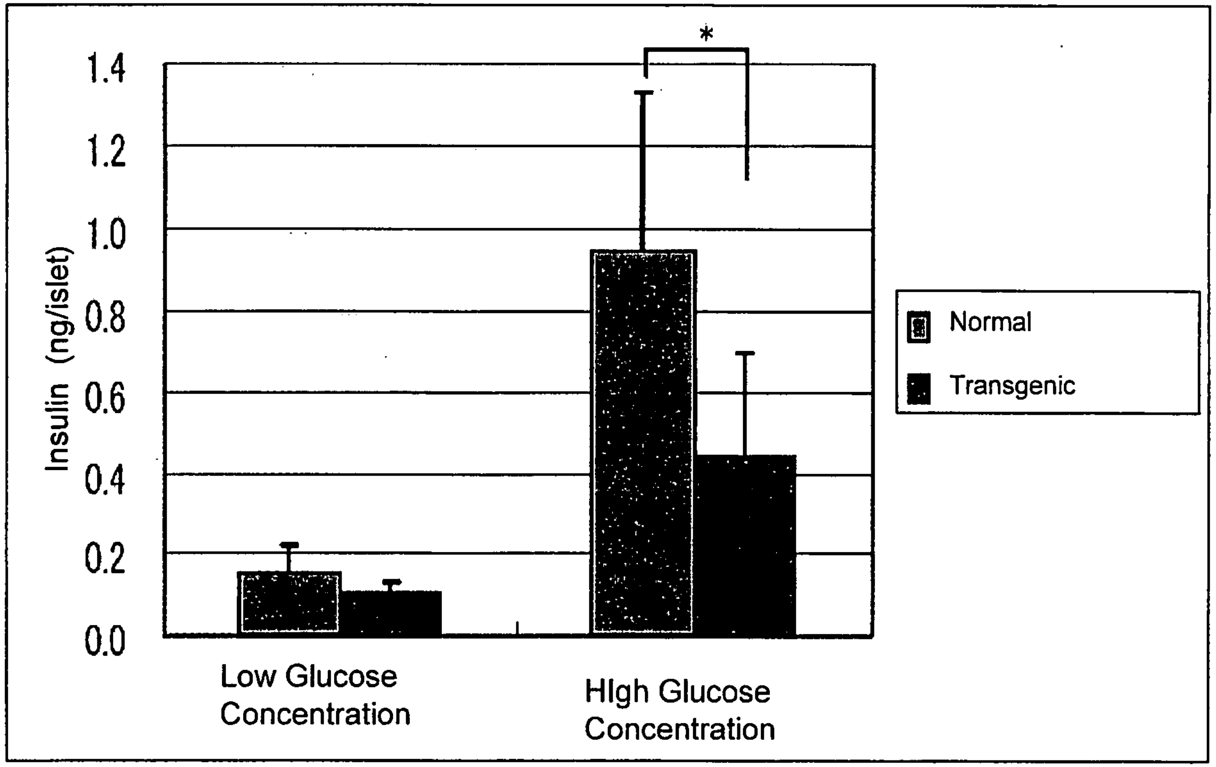


Average value ± SD, n=10 * $p<0.05$ (student t-test)

FIG. 5 ExpressionLevel of Introduced Gene SREBP-2 (hBP-2) and HMG CoA

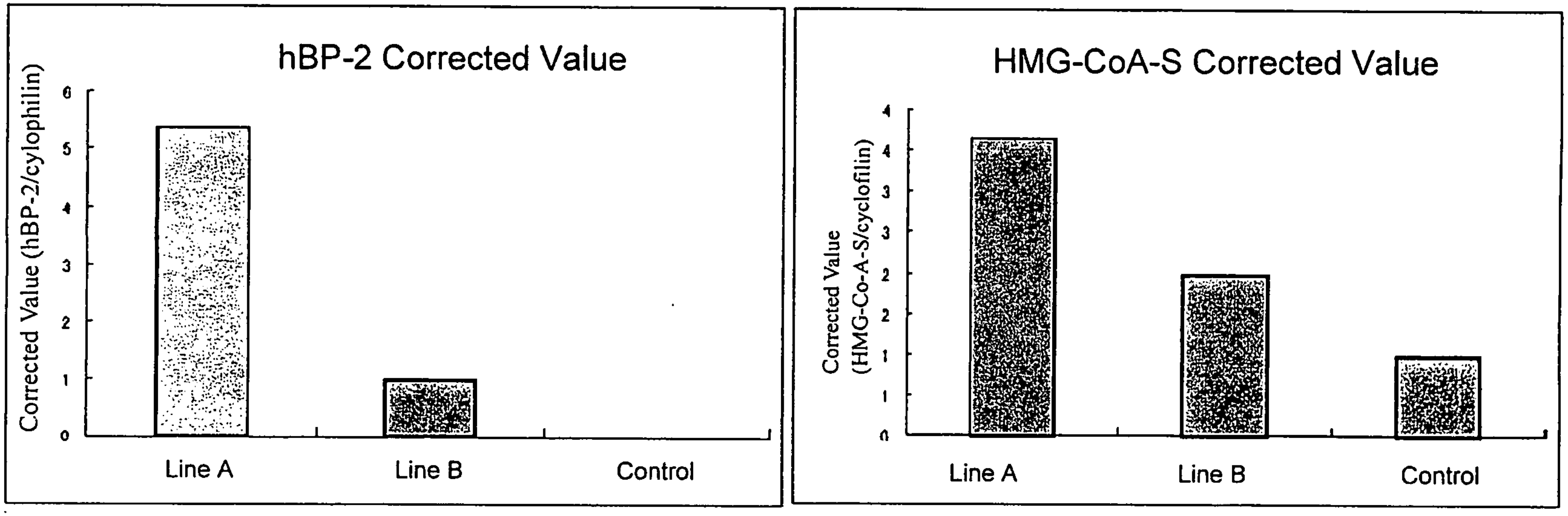

## INTERNATIONAL SEARCH REPORT

International application No.
PCT/JP2005/007285

A. CLASSIFICATION OF SUBJECT MATTER
Int.Cl[7] A01K67/027, 67/02, C12N15/12, 15/65, 15/85, G01N33/50

According to International Patent Classification (IPC) or to both national classification and IPC

B. FIELDS SEARCHED
Minimum documentation searched (classification system followed by classification symbols)
Int.Cl[7] A01K67/027, 67/02, C12N15/12, 15/65, 15/85, G01N33/50

Documentation searched other than minimum documentation to the extent that such documents are included in the fields searched

Electronic data base consulted during the international search (name of data base and, where practicable, search terms used)
WPI(DIALOG), BIOSIS(DIALOG), Medline(STN), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y | Horton J D et al., Activation of cholesterol synthesis in preference to fatty acid synthesis in liver and adipose tissue of transgenic mice overproducing sterol regulatory element-binding protein-2., J.Clin.Invest., 1998 June, Vol.101, No.11, pages 2331 to 2339 | 1-3,7,8,11<br>4-8 |
| X<br>Y | Shinomura I et al., Nuclear sterol regulatory element-binding proteins activate genes responsible for the entire program of unsaturated fatty acid biosynthesis in transgenic mouse liver., J.Biol.Chem., 1998 Dec., Vol.273, No.52, pages 35299 to 35306 | 1-3,7,8,11<br>4-8 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 June, 2005 (15.06.05) | 05 July, 2005 (05.07.05) |
| Name and mailing address of the ISA/<br>Japanese Patent Office<br>Facsimile No. | Authorized officer<br>Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003501102 A
- US 4873191 A


### Non-patent literature cited in the description


- *Science,* 14 November 2003, vol. 302 (5648), 1223-7
- *J. Clin. Invest.,* 1996, vol. 98, 1575-1584
- *J. Clin. Invest.,* 1997, vol. 99, 846-854
- *J. Clin. Invest.,* 1998, vol. 101, 2331-2339
- *Review,* 1997, 331-40
- *Proc. Natl. Acad. Sci. USA,* 15 December 1993, vol. 90 (24), 11603-7
- Guide to Techniques in Mouse Development. Method in Enzymology. Academic Press, Inc, vol. 225
- Up-to-Date Technology of Gene Targetting. Yodo-Sha Co,
- **GORDON, J. W.** Guide to Techniques in Mouse Development. Academic Press, 1993
- Molecular Cloning, A Laboratory Manual. Cold Spring Harbor Press, Cold Spring Harbor Laboratory, 2001
- **ALPERT, S. ; HANAHAN D ; TEITELMAN G.** *Cell,* 22 April 1988, vol. 53 (2), 295-308
- **ALPERT, S. ; HANAHAN, D. ; TEITELMAN, G.** *Cell,* 22 April 1988, vol. 53 (2), 295-308
- **AMEMIYA-KUDO M ; SHIMANO H ; HASTY A H ; YAHAGI N ; YOSHIKAWA T ; MATSUZAKA T ; OKAZAKI H ; TAMURA Y ; IIZUKA Y ; OHASHI K.** *J. Lipid Res.,* 2002, vol. 43 (8), 1220-35
- Guide Techniques in Mouse Development. Method in Enzymology. Academic Press, Inc, vol. 225